# EUROPEAN PATENT APPLICATION

(11) **EP 3 845 176 A1**
(43) Date of publication of application: **07.07.2021**
(21) Application number: 19856181.3
(22) Date of filing: 28.08.2019
(51) Int. Cl.: A61B 18/14

(54) **ELECTRIC SCALPEL HEAD**

(30) Priority: 30.08.2018 JP 2018161682
(71) Applicant: Kyocera Corporation, Kyoto-shi, Kyoto 612-8501 (JP)
(72) Inventor: TODA,Hajime, Kyoto-shi, Kyoto 612-8501 (JP); KOGA,Muneki, Kyoto-shi, Kyoto 612-8501 (JP)
(74) Representative: Viering, Jentschura & Partner mbB Patent- und Rechtsanwälte
(86) International application number: PCT/JP2019/033698
(87) International publication number: WO 2020/045494

(57) **Abstract**

A head for electrical scalpel (10) includes a member (1) that is composed of a ceramic(s), and an electrode rod (2) where at least a part thereof is positioned in the member, wherein a surface that contacts the electrode rod on the member is configured in such a manner that a skewness Rsk that is obtained from a roughness curve is positive, so that the head for electrical scalpel is not readily damaged even if heating and cooling thereof are repeated, and is capable of being used over a long period of time.

## Description

### Field

The present disclosure relates to a head for electrical scalpel.

### Background

An electrical scalpel is used during surgery in order to resect cartilage, a tumor foreign body, and the like of an affected part. Then, an electrical scalpel includes a head for electrical scalpel that is a member that has an electrode rod for causing a high-frequency electric current to flow therethrough. In a case where an electrical scalpel is used, a physiological saline as an electrically conductive fluid is dropped onto an affected part and a high-frequency electric current from an electrode rod that is included in a head for electrical scalpel is caused to flow through such a physiological saline, so that it is possible to resect cartilage, a tumor foreign body, and the like of the affected part.

Herein, it has been known that a ceramic(s) that is/are excellent in a heat resistance is/are used for a member of a head for electrical scalpel (see, for example,

### Patent Literature 1).

### Citation List

### Patent Literature

Patent Literature 1: Japanese Patent Application Publication No. 2002-136526

### Summary

A head for electrical scalpel according to the present disclosure includes a member that is composed of a ceramic(s), and an electrode rod where at least a part thereof is positioned in the member. Then, a surface that contacts the electrode rod on the member is provided in such a manner that a skewness Rsk that is obtained from a roughness curve is positive.

### Brief Description of Drawings

FIG. 1 is a perspective view that schematically illustrates an example of a head for electrical scalpel according to the present disclosure.
FIG. 2 is a cross-sectional view along a line A-A' in FIG. 1.

### Description of Embodiments

Hereinafter, a head for electrical scalpel according to the present disclosure will be explained in detail with reference to the drawing(s).

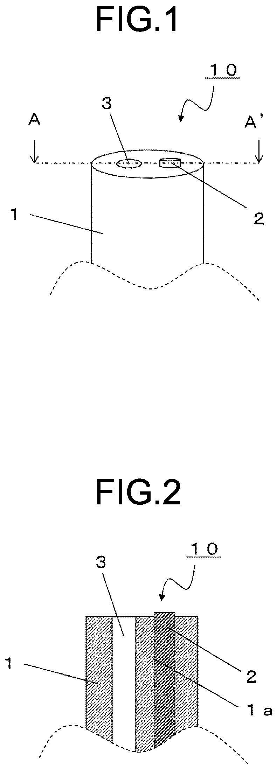

As illustrated in FIG. 1 and FIG. 2, a head for electrical scalpel 10 according to the present disclosure includes a member 1 and an electrode rod 2 where at least a part thereof is positioned in the member 1.

Additionally, although FIG. 1 illustrates a monopolar type head for electrical scalpel 10 that includes one electrode rod 2, this is not limiting and a bipolar type head for electrical scalpel 10 that includes a plurality of electrode rods 2 may be provided.

Furthermore, as illustrated in FIG. 1 and FIG. 2, the head for electrical scalpel 10 according to the present disclosure may include a flow path 3 for suctioning a physiological saline, blood, a fine tissue that is resected from an affected part, or the like.

Herein, the ember 1 is composes of a ceramic(s). A ceramic(s) is/are an aluminum-oxide-based ceramic(s), a zirconium-oxide-based ceramic(s), a silicon-nitride-based ceramic(s), an aluminum-nitride-based ceramic(s), a silicon-carbide-based ceramic(s), a mullite-based ceramic(s), or the like.

Thus, the member 1 is composed of a ceramic(s), and hence, is excellent in a heat resistance. In particular, if the member 1 is composed of a silicon-nitride-based ceramic(s) among ceramics, it is of a high thermal conductivity and is excellent in a mechanical strength as well as a heat resistance.

Herein, a silicon-nitride-based ceramic(s) contain(s) 70% by mass or more of silicon nitride among 100% by mass of all components that compose the ceramic(s). Then, it is possible to confirm a material of the member 1 by an undermentioned method. First, the member 1 is measured by using an X-ray diffractometer (XRD) and a value of an obtained 2θ (where 2θ is a diffraction angle) is identified by a JCPDS card. Then, quantitative analysis of each component that composes the member 1 is executed by using an ICP (Inductively Coupled Plasma) emission spectrophotometer (ICP) or an X-ray fluorescence spectrometer (XRF). Herein, if presence of silicon nitride is confirmed in an XRD and a content of silicon nitride (Si₃N₄) that is converted from a content of silicon (Si) that is measured by an ICP or an XRF is 70% by mass or more, it is a silicon-nitride-based ceramic(s).

Furthermore, the electrode rod 2 may be composed of any material as long as it has an electrically conductive property, and may be composed of, for example, tungsten, titanium, or molybdenum. If the electrode rod 2 is thus composed of tungsten, titanium, or molybdenum, it is not harmful for an affected part and has a high heat resistance, so that it is suitable for use over a long period of time.

Then, in the head for electrical scalpel 10 according to the present disclosure, a surface 1a that contacts the electrode rod 2 on the member 1 (that will also be described as, simply, a surface 1a below) is provided in such a manner that a skewness Rsk that is obtained from a roughness curve is positive.

Herein, a skewness Rsk that is obtained from a roughness curve is defined in JIS B 0601 (2013) and is an index that indicates, when a mean height of a roughness curve is provided as a center line, a ratio of a peak and a valley with respect thereto. Then, if a skewness Rsk is positive, it is indicated that a slope to a deepest part of a valley is gentler than that in a case where a skewness Rsk is negative.

Although a crack is readily generated from a bottom of a valley of the surface 1a as heating and cooling thereof are repeated, the head for electrical scalpel 10 according to the present disclosure satisfies such a configuration where a slope to a deepest part of a valley of the surface 1a is gentler than that in a case where a skewness Rsk is negative, so that a crack is not readily generated and use thereof over a long period of time is possible.

Furthermore, the surface 1a in the head for electrical scalpel 10 according to the present disclosure may be provided in such a manner that a ratio Rvk/(Rvk+Rk+Rpk) of a reduced valley depth Rvk that is obtained from a roughness curve, a core roughness depth Rk that is obtained from a roughness curve, and a reduced peak height Rpk that is obtained from a roughness curve is 0.4 or less.

Herein, a reduced valley depth Rvk, a core roughness depth Rk, and a reduced peak height Rpk are defined in JIS B 0671-2 (2002) and are provided by an undermentioned definition. First, a straight line that is provided in such a manner that a secant line of a material ratio curve that is drawn in such a manner that a material ratio difference is 40%, at a central part of the material ratio curve that includes 40% of measurement points on a roughness curve, is of a gentlest slope is provided as an equivalent line. Then, a part between two height positions where such an equivalent line intersects with a longitudinal axis at positions where material ratios are 0% and 100% is provided as a core. Then, a core roughness depth Rk is an index that indicates a level difference between an upper side and a lower side of such a core. Furthermore, for a roughness curve, a reduced valley depth Rvk is a mean depth of a projecting valley that is present below a core and a reduced peak height Rpk is a mean height of a projecting peak that is present above the core.

Then, if such a configuration is satisfied, a depth of a projecting valley on the surface 1a is small, and a crack is not readily generated from a bottom of a projecting valley even if heating and cooling thereof are repeated, so that the head for electrical scalpel 10 according to the present disclosure is further prevented from being readily damaged.

Furthermore, the surface 1a in the head for electrical scalpel 10 according to the present disclosure may be provided in such a manner that a reduced valley depth Rvk is 2 µm or less. If such a configuration is satisfied, a depth of a projecting valley on the surface 1a is smaller, and a crack is not readily generated from a bottom of a projecting valley even if heating and cooling thereof are repeated, so that the head for electrical scalpel 10 according to the present disclosure is further prevented from being readily damaged.

Herein, a lower limit of a reduced valley depth Rvk on the surface 1a of the member 1 that is composed of a ceramic(s) is, for example, 0.2 µm, from the viewpoint of such a reduced valley depth Rvk that is substantially not 0 µm. Furthermore, a core roughness depth Rk on the surface 1a is, for example, 0.3 µm or greater and 6 µm or less. Furthermore, a reduced peak height Rpk on the surface 1a is, for example, 0.05 µm or greater and 3 µm or less.

Furthermore, the surface 1a in the head for electrical scalpel 10 according to the present disclosure may be provided in such a manner that a mean spacing of profile irregularities Rsm that is obtained from a roughness curve is 15µm or greater. Herein, a mean spacing of profile irregularities Rsm is defined in JIS B 0601 (2013) and is an index that indicates, when a sum of a length of a center line that corresponds to one peak and one valley that is adjacent thereto is provided as a spacing between a peak and a valley, a mean value of such a spacing.

If such a configuration is satisfied, the number of a large valley(s) on the surface 1a is small, and a possibility of generating a crack from a valley is small even if heating and cooling thereof are repeated, so that the head for electrical scalpel 10 according to the present disclosure is further prevented from being readily damaged. Herein, if an upper limit of a mean spacing of profile irregularities Rsm on the surface 1a is set, it is, for example, 80µm or less.

Furthermore, the surface 1a in the head for electrical scalpel 10 according to the present disclosure may be provided in such a manner that a mean spacing of local peaks S that is obtained from a roughness curve is 8µm or greater. Herein, a mean spacing of local peaks S is defined in JIS B 0601 (1994), and is an index that indicates a mean value of a spacing of both adjacent local peaks.

Then, if such a configuration is satisfied, the number of not only a large valley(s) on the surface 1a but also a small valley(s) thereon is small, and a possibility of generating a crack from a valley is small even if heating and cooling thereof are repeated, so that the head for electrical scalpel 10 according to the present disclosure is further prevented from being readily damaged. Herein, if an upper limit of a mean spacing of local peaks S on the surface 1a is set, it is, for example, 30µm or less.

Herein, it is possible to measure and thereby obtain a skewness Rsk and a mean spacing of profile irregularities Rsm on the surface 1a in the head for electrical scalpel 10 according to the present disclosure in conformity with JIS B 0601 (2013), a reduced valley depth Rvk, a core roughness depth Rk, and a reduced peak height Rpk in conformity with JIS B 0671-2 (2002), and a mean spacing of local peaks S in conformity with JIS B 0601 (1994). Additionally, for a measurement condition(s), it is sufficient to set, for example, a measurement length at 4.8 mm, a cutoff value at 0.8 mm, and a scanning rate of a probe with a probe radius of 2 µm at 1.0 mm/sec. Then, it is sufficient that at least 3 or more points on the surface 1a that is exposed by executing cutting or the like of the head for electrical scalpel 10 are measured and a mean value thereof is obtained.

Hereinafter, a manufacturing method for a head for electrical scalpel 10 according to the present disclosure will be explained. Additionally, an explanation is herein provided by providing, as an example, a case where a member 1 therein is composed of a silicon-nitride-based ceramic(s).

First, a silicon nitride (Si₃N₄) powder as a main raw material, and an yttrium oxide (Y₂O₃) powder and an aluminum oxide (Al₂O₃) powder as sintering aids, together with a solvent and a ball(s), are put into a mill, and are pulverization thereof is executed so as to provide a predetermined particle size, so that a slurry is fabricated. Additionally, a calcium oxide (CaO) powder, a ferric oxide (Fe₂O₃) powder, a tungsten oxide (WO₃) powder, and the like, other than an yttrium oxide powder and an aluminum oxide powder, may be added as sintering aids.

Then, a binder is added to an obtained slurry and subsequently spray drying is executed by using a spray dryer, so that a granule(s) is/are fabricated.

Then, such a granule(s), a thermoplastic resin, a wax, and the like are thrown into a kneader and are kneaded while heating is executed, so that a clay is obtained.
Then, an obtained clay is thrown into a pelletizer, so that a pellet that is a raw material for injection forming (injection molding) is obtained. Then, an obtained pellet is thrown into an injection molder (injection molding machine) and injection molding is executed, so that obtain a molded body that has a hole for inserting an electrode rod 2 is obtained. Additionally, a through-hole that provides a flow path 3 may be formed on a molded body.

Thus, in order to obtain a molded body that has a hole for inserting an electrode rod 2, it is sufficient that a molded tool that is capable of obtaining a hole is fabricated based on a general injection molding method and this is placed in an injection molder where injection molding is executed. Then, a surface texture at a place where a hole is formed on such a molding tool is transferred to an inner surface of the hole, so that it is sufficient that a molded body is fabricated by using a molding tool that has a surface texture that corresponds thereto, in order that a skewness Rsk on a surface 1a that contacts the electrode rod 2 in the member 1 is positive. Additionally, a case where a reduced valley depth Rvk, a core roughness depth Rk, a reduced peak height Rpk, a mean spacing of profile irregularities Rsm, and a mean spacing of local peaks S on the surface 1a are any values is also similar thereto.

Then, an obtained molded body is held and fired in a nitrogen gas atmosphere at a maximum temperature of 1700°C or higher and 1800°C or lower for 0.5 hours or more and 4 hours or less, so that a sintered body is obtained. Then, a surface of an obtained sintered body is barrel-polished. Additionally, a firing condition(s) is/are changed depending on a shape and a size of a product, so that it is sufficient that adjustment thereof is executed as needed.

Then, if the electrode rod 2 is fitted into a hole of a sintered body, it is possible to obtain the head for electrical scalpel 10 according to the present disclosure.

Additionally, the present disclosure is not limited to an embodiment(s) as described above and a variety of modifications, improvements, or the like are possible without departing from a spirit of the present disclosure.

### Reference Signs List

- 1:: member
- 1a:: surface that contacts an electrode rod on a member
- 2:: electrode rod
- 3:: flow path
- 10:: head for electrical scalpel

## Claims

1. A head for electrical scalpel, comprising:
a member that is composed of a ceramic(s); and
an electrode rod where at least a part thereof is positioned in the member, wherein
a surface that contacts the electrode rod on the member is provided in such a manner that a skewness Rsk that is obtained from a roughness curve is positive.

2. The head for electrical scalpel according to claim 1, wherein
the surface is provided in such a manner that a ratio Rvk/(Rvk+Rk+Rpk) of a reduced valley depth Rvk that is obtained from a roughness curve, a core roughness depth Rk that is obtained from a roughness curve, and a reduced peak height Rpk that is obtained from a roughness curve is 0.4 or less.

3. The head for electrical scalpel according to claim 1 or claim 2, wherein
the surface is provided in such a manner that a reduced valley depth Rvk that is obtained from a roughness curve is 2 µm or less.

4. The head for electrical scalpel according to any of claim 1 to claim 3, wherein
the surface is provided in such a manner that a mean spacing of profile irregularities Rsm that is obtained from a roughness curve is 15 µm or greater.

5. The head for electrical scalpel according to any of claim 1 to claim 4, wherein
the surface is provided in such a manner that a mean spacing of local peaks S that is obtained from a roughness curve is 8 µm or greater.

6. The head for electrical scalpel according to any of claim 1 to claim 5, wherein
the member is composed of a silicon-nitride-based ceramic(s).
